(19) Europäisches Patentamt
European Patent Office
Office européen des brevets

(11) Veröffentlichungsnummer: **0 041 242**
**B1**

(12) **EUROPÄISCHE PATENTSCHRIFT**

(45) Veröffentlichungstag der Patentschrift: **11.07.84**

(51) Int. Cl.³: **C 07 D 243/20**, C 07 D 401/04, A 61 K 31/55

(21) Anmeldenummer: **81104102.9**

(22) Anmeldetag: **28.05.81**

(54) **Carbamoyloxyamino-1,4-benzodiazepine, Verfahren zu ihrer Herstellung und diese enthaltende Arzneimittel.**

(30) Priorität: **04.06.80 DE 3021107**

(43) Veröffentlichungstag der Anmeldung: **09.12.81 Patentblatt 81/49**

(45) Bekanntmachung des Hinweises auf die Patenterteilung: **11.07.84 Patentblatt 84/28**

(84) Benannte Vertragsstaaten: **AT BE CH DE FR GB IT LI LU NL SE**

(56) Entgegenhaltungen:
**FR - A - 2 073 379**
**US - A - 3 857 854**

**JOURNAL OF MEDICINAL CHEMISTRY, Band 16, Nr. 11, 1973 R.B. MOFFETT et al.: "Central Nervous System Depressants. II. Benzodiazepines with Ureas in the 2 Position", Seiten 1256-1257**

(73) Patentinhaber: **HOECHST AKTIENGESELLSCHAFT, Postfach 80 03 20, D-6230 Frankfurt am Main 80 (DE)**

(72) Erfinder: **Försch, Manfred, Dr., Kranichstrasse 66, D-6085 Nauheim (DE)**
Erfinder: **Gerhards, Hermann, Dr., Wacholderweg 4, D-6238 Hofheim am Taunus (DE)**

EP 0 041 242 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine neue Klasse von Carbamoyloxyaminoverbindungen, die eine angstlösende Wirkung besitzen ohne zu sedieren, Verfahren zur Herstellung dieser neuen Verbindungen sowie pharmazeutische Mittel, welche die erfindungsgemässen aktiven Verbindungen enthalten.

Gegenstand der Erfindung sind 1,4-Benzodiazepine mit einer Carbamoyloxyaminogruppierung der Formel I

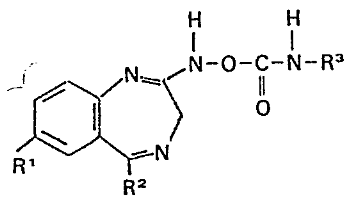

(I)

und die tautomeren Verbindungen der Formel IA

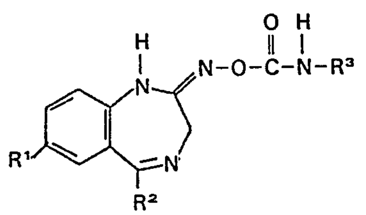

(IA)

und deren physiologisch verträgliche Salze, worin

$R^1$ ein Halogenatom oder eine Nitrogruppe ist,

$R^2$ Phenyl oder ein durch Halogen einfach substituierter Phenylring ist, und

$R^3$ Phenyl oder ein durch Halogen oder Trifluormethyl einfach substituierter Phenylring ist, wobei Halogen jeweils bevorzugt Fluor, Chlor oder Brom bedeutet.

Die erfindungsgemässen Verbindungen der Formeln I und IA können hergestellt werden, indem man

a) eine Verbindung der Formel II

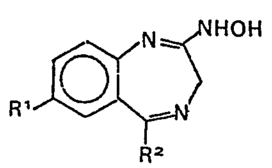

(II)

worin $R^1$ und $R^2$ die obengenannte Bedeutung haben, mit einem Isocyanat der Formel III

$$O=C=N-R^3 \qquad (III)$$

oder einem reaktionsfähigen Carbaminsäurederivat der Formel IV

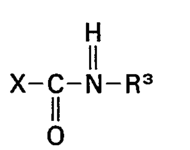

(IV)

umsetzt, worin X für Halogen oder den Phenoxyrest steht und $R^3$ die obengenannte Bedeutung hat;

b) eine Verbindung der Formel V

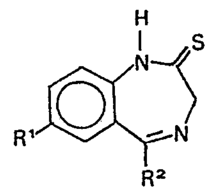

(V)

worin $R^1$ und $R^2$ die obengenannte Bedeutung besitzen, mit Hydroxylamin bzw. einem Salz des Hydroxylamins, aus dem während der Reaktion die Base freigesetzt wird, zur Reaktion bringt und anschliessend mit einem Isocyanat der Formel III oder einem Carbaminsäurederivat der Formel IV umsetzt; oder

c) eine Verbindung der Formel V mit einem O-acylierten Hydroxylamin der Formel VI

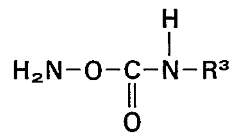

(VI)

worin $R^3$ die obengenannte Bedeutung hat, umsetzt.

Die Herstellung der erfindungsgemässen Verbindungen gemäss Formel I nach Verfahren a aus einer Verbindung der allgemeinen Formel II und einem Isocyanat wird bei einer Temperatur von 0 bis 120, vorzugsweise bei 20 bis 80° C, in einem inerten aprotischen Lösungsmittel vorzugsweise Ether, wie Dioxan, Tetrahydrofuran, 1,2-Dimethoxyethan, Diethylenglykoldimethylether oder Dimethylformamid, Dimethylsulfoxid oder Hexamethylphosphorsäuretriamid durchgeführt. Nach beendeter Umsetzung wird abfiltriert und das Produkt durch Zusatz von kaltem Wasser zum Reaktionsgemisch ausgefällt. Gegebenenfalls wird vorher das Lösungsmittel im Vakuum abgedampft und dann erst mit Wasser versetzt. Das Rohprodukt kann durch Umkristallisation aus einem Lösungsmittel gereinigt werden. Die Umsetzung von Verbindungen der allgemeinen Formel II mit Carbaminsäurehalogeniden der Formel IV wird in einem inerten Lösungsmittel vorzugsweise in Toluol, Dioxan, Tetrahydrofuran, 1,2-Dimethoxyethan, Dimethylformamid, Chloroform oder Methylenchlorid durchgeführt. Gegebenenfalls ist die Zugabe von Hilfsbasen, z.B. Alkalimetallhydroxiden, Alkalimetallalkoholaten, heterocyclischen Stickstoffbasen wie Pyridin oder tertiären Aminen vorzugsweise Triethylamin oder Ethyldiisopropylamin vorteilhaft.

Die Reaktion wird bei −10 bis 60, vorzugsweise bei 0° C bis Raumtemperatur, durchgeführt.

Die Umsetzung von Verbindungen der Formel II mit Carbaminsäurephenylester wird in Alkoholen vorzugsweise in Methanol durchgeführt. Die Reaktionstemperatur liegt zwischen Raumtemperatur und dem Siedepunkt des jeweiligen Lösungsmittels.

Bei Verfahren b wird eine Verbindung der Formel V mit einer Hydroxylaminlösung in einem inerten aprotischen Lösungsmittel wie in Verfahren a angegeben umgesetzt. Gegebenenfalls kann das Hydroxylamin *in situ* aus einem Hydroxylaminsäureadditionssalz durch Zusatz von säurebindenden Mitteln wie z.B. Alkalimetallhydroxide, -carbonate, -hydrogencarbonate, -alkanolate, heterocyclische Stickstoffbasen, wie Pyridin, oder tertiäre Amine, vorzugsweise Alkalimetallhydrogencarbonate, insbesondere Natriumhydrogencarbonat freigesetzt werden.

Die Umsetzung kann bei Raumtemperatur oder mässig erhöhten Temperaturen bis 60° C durchgeführt werden. Die Reaktionszeit beträgt bei Raumtemperatur 2 bis 24 h, vorzugsweise lässt man den Reaktionsansatz über Nacht bei Raumtemperatur stehen.

Vor Zugabe des Isocyanats der Formel III wird abfiltriert und wie unter Verfahren a angegeben aufgearbeitet.

Bei Verfahren c wird die Thioverbindung der Formel V mit einem O-acylierten Hydroxylamin der Formel VI in einem Lösungsmittel vorzugsweise Alkohol, Methanol oder Isopropanol umgesetzt. Auch die Verwendung von Dimethylformamid, Dioxan, Tetrahydrofuran oder Diethylenglykoldimethylether kann vorteilhaft sein. Man arbeitet dabei, je nach Substituenten $R^3$ bei −10 bis 60, vorzugsweise bei 0° C bis Zimmertemperatur.

Die O-acylierten Hydroxylamine der Formel VI lassen sich nach G. Zinner und Mitarbeiter, „Archiv der Pharmazie", Bd. *303*, S. 317 (1970) aus den entsprechenden O-acylierten Acethydroximsäureethylester darstellen:

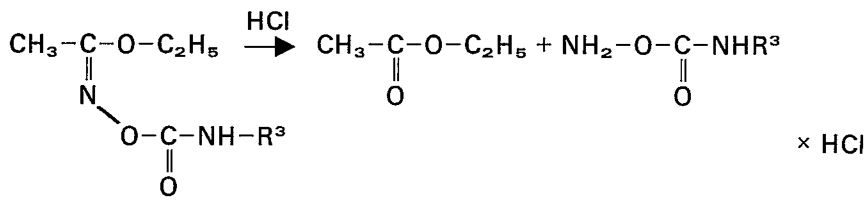


(VI)

Die O-acylierten Hydroxylamine der Formel VI werden vorzugsweise in Form ihrer Salze, insbesondere ihrer Hydrochloride, eingesetzt. Als säurebindende Mittel für Umsetzungen dieser O-acylierten Hydroxylamine können z.B. Alkalimetallhydroxide, -carbonate, -hydrogencarbonate, -alkoholate, heterocyclische Stickstoffbasen, wie Pyridin, oder tertiäre Amine, vorzugsweise Alkalimetallhydrogencarbonate, insbesondere Natriumhydrogencarbonat, verwendet werden.

Die Synthese der Ausgangsstoffe der Formel II ist zwar prinzipiell in den DE-OS Nrn. 2005176 und 2135595 sowie US Nr. 3857354 beschrieben, lässt sich aber teilweise nicht nachvollziehen.

In diesen Arbeiten werden die Verbindungen der Formel II aus den Thiolactamen der Formel V durch Umsetzung mit Hydroxylammoniumchlorid und Natriumbicarbonat in siedendem Methanol bei 2stündiger Reaktionsdauer hergestellt. Wir haben festgestellt, dass bei diesen Bedingungen Nebenprodukte in grosser Menge anfallen, die nur durch eine aufwendige säulenchromatographische Reinigung abgetrennt werden können. Darüber hinaus erweisen sich Verbindungen der allgemeinen Formel II als säureempfindlich.

Wir haben nun eine Methode gefunden, wie sie in Verfahren b beschrieben wird. Dabei werden Verbindungen der Formel V mit Hydroxylamin oder einem Salz desselben, vorzugsweise in Dioxan/Dimethylformamid, wenn nötig unter Zusatz von Natriumhydrogencarbonat zur Reaktion gebracht und Anschliessend mit einem Isocyanat der Formel III versetzt. Die Verbindungen der allgemeinen Formel II werden dabei nicht isoliert.

Besonders glatt verläuft die Darstellung der Verbindungen der Formel II, wenn frisch kristallisiertes Hydroxylamin nach H. Lecher und J. Hofmann in „Chem. Ber.", 55, S. 912 (1922) in die Reaktion eingesetzt wird.

Die Ausgangsstoffe der Formel V, soweit sie nicht in der Literatur beschrieben sind (G. Archer und L.H. Sternbach, „J. Org. Chem.", Bd. *20*, S. 231 (1964); US-PS Nr. 3422091), werden durch Umsetzung der bekannten Amide der allgemeinen Formel VII, worin $R^1$ und $R^2$ die zur Formel I genannten Bedeutungen haben

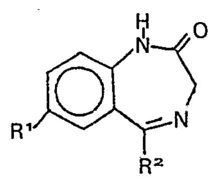

(VII)

mit Phosphorpentasulfid in Pyridin bei 100° C, oder mit dem dimeren p-Methoxyphenylthionophosphinsulfid in Dioxan nach S.-O. Lawesson in „Bull. Soc. Chim. Belg.", *87*, 229 (1978) hergestellt.

Die Erfindung betrifft auch diejenigen Ausführungsformen der Verfahren, bei denen man von einer auf irgendeiner Stufe des Verfahrens als Zwischenprodukt erhältlichen Verbindung ausgeht und die zusätzlichen Verfahrensschritte durchführt, oder das Verfahren auf irgendeiner Stufe abbricht, oder eine als Ausgangsstoff verwendete Verbindung unter den Reaktionsbedingungen bildet oder in Form eines reaktionsfähigen Derivates oder Salzes verwendet.

Als erfindungsgemässe Verbindungen können ausserdem in den Beispielen genannten vorzugsweise die folgenden hergestellt werden:

7-Brom-2-(3-chlorphenylcarbamoyloxyamino)-5-phenyl-3H-1,4-benzodiazepin

7-Brom-2-(3-fluorphenylcarbamoyloxyamino)-5-phenyl-3H-1,4-benzodiazepin

7-Brom-2-(3-bromphenylcarbamoyloxyamino)-5-phenyl-3H-1,4-benzodiazepin

7-Brom-2-(3-trifluormethylphenylcarbamoyloxyamino)-5-phenyl-3H-1,4-benzodiazepin

7-Brom-2-(4-chlorphenylcarbamoyloxyamino)-5-phenyl-3H-1,4-benzodiazepin

7-Brom-2-(3-chlorphenylcarbamoyloxyamino)-5-(2-fluorphenyl)-3H-1,4-benzodiazepin
7-Brom-2-(3-fluorphenylcarbamoyloxyamino)-5-(2-fluorphenyl)-3H-1,4-benzodiazepin
7-Brom-2-(3-bromphenylcarbamoyloxyamino)-5-(2-fluorphenyl)-3H-1,4-benzodiazepin
7-Brom-2-(3-trifluormethylphenylcarbamoyloxyamino)-5-(2-fluorphenyl)-3H-1,4-benzodiazepin
7-Brom-2-(4-chlorphenylcarbamoyloxyamino)-5-(2-fluorphenyl)-3H-1,4-benzodiazepin
7-Brom-2-(3-chlorphenylcarbamoyloxyamino)-5-(2-chlorphenyl)-3H-1,4-benzodiazepin
7-Brom-2-(3-fluorphenylcarbamoyloxyamino)-5-(2-chlorphenyl)-3H-1,4-benzodiazepin
7-Brom-2-(3-bromphenylcarbamoyloxyamino)-5-(2-chlorphenyl)-3H-1,4-benzodiazepin
7-Brom-2-(3-trifluormethylphenylcarbamoyloxyamino)-5-(2-chlorphenyl)-3H-1,4-benzodiazepin
7-Brom-2-(4-chlorphenylcarbamoyloxyamino)-5-(2-chlorphenyl)-3H-1,4-benzodiazepin
7-Brom-2-(3-chlorphenylcarbamoyloxyamino)-5-(2-bromphenyl)-3H-1,4-benzodiazepin
7-Brom-2-(3-fluorphenylcarbamoyloxyamino)-5-(2-bromphenyl)-3H-1,4-benzodiazepin
7-Brom-2-(3-bromphenylcarbamoyloxyamino)-5-(2-bromphenyl)-3H-1,4-benzodiazepin
7-Brom-2-(3-trifluorphenylcarbamoyloxyamino)-5-(2-bromphenyl)-3H-1,4-benzodiazepin
7-Brom-2-(4-chlorphenylcarbamoyloxyamino)-5-(2-bromphenyl)-3H-1,4-benzodiazepin
7-Chlor-2-(3-chlorphenylcarbamoyloxyamino)-5-phenyl-3H-1,4-benzodiazepin
7-Chlor-2-(3-fluorphenylcarbamoyloxyamino)-5-phenyl-3H-1,4-benzodiazepin
7-Chlor-2-(3-bromphenylcarbamoyloxyamino)-5-phenyl-3H-1,4-benzodiazepin
7-Chlor-2-(3-trifluormethylphenylcarbamoyloxyamino)-5-phenyl-3H-1,4-benzodiazepin
7-Chlor-2-(4-chlorphenylcarbamoyloxyamino)-5-phenyl-3H-1,4-benzodiazepin
7-Chlor-2-(3-chlorphenylcarbamoyloxyamino)-5-(2-fluorphenyl)-3H-1,4-benzodiazepin
7-Chlor-2-(3-fluorphenylcarbamoyloxyamino)-5-(2-fluorphenyl)-3H-1,4-benzodiazepin
7-Chlor-2-(3-bromphenylcarbamoyloxyamino)-5-(2-fluorphenyl)-3H-1,4-benzodiazepin
7-Chlor-2-(3-trifluorphenylcarbamoyloxyamino)-5-(2-fluorphenyl)-3H-1,4-benzodiazepin
7-Chlor-2-(4-chlorphenylcarbamoyloxyamino)-5-(2-fluorphenyl)-3H-1,4-benzodiazepin
7-Chlor-2-(3-chlor-4-methylphenylcarbamoyloxyamino)-5-(2-fluorphenyl)-3H-1,4-benzodiazepin
7-Chlor-2-(3-chlorphenylcarbamoyloxyamino)-5-(2-chlorphenyl)-3H-1,4-benzodiazepin
7-Chlor-2-(3-fluorphenylcarbamoyloxyamino)-5-(2-chlorphenyl)-3H-1,4-benzodiazepin
7-Chlor-2-(3-bromphenylcarbamoyloxyamino)-5-(2-chlorphenyl)-3H-1,4-benzodiazepin
7-Chlor-2-(3-trifluormethylphenylcarbamoyloxyamino)-5-(2-chlorphenyl)-3H-1,4-benzodiazepin
7-Chlor-2-(4-chlorphenylcarbamoyloxyamino)-5-(2-chlorphenyl)-3H-1,4-benzodiazepin
7-Nitro-2-(3-chlorphenylcarbamoyloxyamino)-5-phenyl-3H-1,4-benzodiazepin
7-Nitro-2-(3-fluorphenylcarbamoyloxyamino)-5-phenyl-3H-1,4-benzodiazepin
7-Nitro-2-(3-bromphenylcarbamoyloxyamino)-5-phenyl-3H-1,4-benzodiazepin
7-Nitro-2-(3-trifluormethylphenylcarbamoyloxyamino)-5-phenyl-3H-1,4-benzodiazepin
7-Nitro-2-(4-chlorphenylcarbamoyloxyamino)-5-phenyl-3H-1,4-benzodiazepin
7-Nitro-2-(3-chlorphenylcarbamoyloxyamino)-5-(2-chlorphenyl)-3H-1,4-benzodiazepin
7-Nitro-2-(3-fluorphenylcarbamoyloxyamino)-5-(2-chlorphenyl)-3H-1,4-benzodiazepin
7-Nitro-2-(3-bromphenylcarbamoyloxyamino)-5-(2-chlorphenyl)-3H-1,4-benzodiazepin
7-Nitro-2-(3-trifluormethylphenylcarbamoyloxyamino)-5-(2-chlorphenyl)-3H-1,4-benzodiazepin
7-Nitro-2-(4-chlorphenylcarbamoyloxyamino)-5-(2-chlorphenyl)-3H-1,4-benzodiazepin
7-Nitro-2-(3-chlorphenylcarbamoyloxyamino)-5-(2-fluorphenyl)-3H-1,4-benzodiazepin
7-Nitro-2-(3-fluorphenylcarbamoyloxyamino)-5-(2-fluorphenyl)-3H-1,4-benzodiazepin
7-Nitro-2-(3-bromphenylcarbamoyloxyamino)-5-(2-fluorphenyl)-3H-1,4-benzodiazepin
7-Nitro-2-(3-trifluormethylphenylcarbamoyloxyamino)-5-(2-fluorphenyl)-3H-1,4-benzodiazepin
7-Nitro-2-(4-chlorphenylcarbamoyloxyamino)-5-(2-fluorphenyl)-3H-1,4-benzodiazepin

Die Verbindungen der Formel I können nach den beschriebenen Verfahren als solche oder in Form eines Säureadditionssalzes isoliert werden. Bei den Salzen handelt es sich vorzugsweise um die pharmazeutisch verträglichen, nichttoxischen Additionssalze mit geeigneten Säuren, wie z.B. solchen mit anorganischen Säuren, wie Chlorwasserstoff-, Bromwasserstoff- und Jodwasserstoff-, Salpeter-, Schwefel- oder Phosphorsäure oder mit organischen Säuren, wie Ameisen-, Essig-, Propion-, Bernstein-, Wein-, Malein-, Glykol-, Milch-, Hydroxymalein-, Fumar-, Oxal-, Zitronen-, Apfel-, Schleim-, Benzoe-, Salicyl-, Acetur-, Embon-, Naphthalin-1,5-disulfon-, Ascorbin-, Phenylessig-, p-Aminosalicyl-, Hydroxyethansulfon-, Benzolsulfonsäure oder synthetische Harze, die saure Gruppen enthalten, z.B. solche mit Ionenaustauscherwirkung.

Das dabei erhaltene Säureadditionssalz kann nach bekannten Verfahren in die freie Verbindung umgewandelt werden, beispielsweise durch Behandlung desselben mit einer Base, wie einem Metallhydroxid, Metallalkoholat, Metallcarbonat, mit Ammoniak oder mit einem Hydroxylionenaustauscher oder mit irgendeinem anderen geeigneten Reagens. Ein erhaltenes Säureadditionssalz kann nach bekannten Verfahren in ein anderes Salz umgewandelt werden; so kann beispielsweise ein Salz mit einer anorganischen Säure mit einem Metallsalz, wie z.B. einem Natrium-, Barium-, oder Silbersalz, einer Säure in einem geeigneten

Verdünnungsmittel, in dem das erhaltene anorganische Salz unlöslich ist, behandelt und dadurch aus dem Reaktionsmedium entfernt werden. Ein Säureadditionssalz kann auch durch Behandlung mit einem Anionenaustauscherpräparat in ein anderes Säureadditionssalz umgewandelt werden. Ein quartäres Ammoniumsalz kann durch Umsetzung der freien Base mit einem Alkylhalogenid hergestellt werden.

In der DE-OS Nr. 2533575 werden Laktamoximcarbamate mit Wirkung auf den Kreislauf beschrieben, während in den DE-OS Nrn. 2005176 und 2135595 und dem US-PS Nr. 3857854 Oxyamino-3H-1,4-benzodiazepin beansprucht werden, die eine angstlösende, mit Sedierung verbundene Wirkung besitzen. Es war aber nicht zu erwarten, dass die noch nicht beschriebenen erfindungsgemässen Carbamoyloxyamino-3H-1,4-benzodiazepine der allgemeinen Formel I einen so dramatischen Split zwischen Anxiolyse und Sedierung zeigen. In den zu verwendenten therapeutischen Dosen tritt praktisch keine Sedierung ein.

Die erfindungsgemässen Verbindungen und ihre pharmakologisch verträglichen Salze werden im Geller-Konflikt-Test *(Punishment Discrimination)* an der Ratte untersucht. (Literatur dazu: I. Geller *et al.*, „Psychopharmacologia", Bd. III, S. 374-385 [1962].)

Weibliche Wistar-Ratten im Gewicht von 100 bis 120 g erlernen zuerst in einer sogenannten Skinner-Box, eine Taste für eine Milchbelohnung zu drücken. Nach einer gewissen Anlernphase wird die Häufigkeit der Milchbelohnung pro Tastendruckrate allmählich reduziert. Hierzu kommt noch eine Tonphase, in der die Belohnung mit einem leichten elektrischen Fussschock über das Bodengitter gekoppelt ist.

Nach durchschnittlich 6- bis 8-Wochen-Training haben die meisten Tiere das Trainingskriterium erreicht, nämlich kontinuierliches Tasten in der schockfreien Phase mit 100 bis 200 Tastdrücke in 10 min sowie Herabsetzung der Tastendruckrate in der Schockphase bis auf 4 bis 5 Tastendrücke in 3 min.

Die erfindungsgemässen Verbindungen werden nun ½ h, bevor die Tiere in die Skinner-Boxen gesetzt werden, *p.o.* verabreicht. Eine Zunahme der Tastendrucke in der Schockphase gegenüber den Trainingswerten (vom Vortage) werden als Zeichen einer anxiolytischen Wirkung der Prüfsubstanz gewertet, während eine Verringerung der Tastendruckrate in der schockfreien Phase als Zeichen einer sedierenden Wirkung des Präparates gewertet wird. Dis Tastraten unter Präparateeinfluss werden in Prozent der Kontrollwerte angegeben, eine Signifikanzberechnung erfolgt mit einem nicht parametrischen Test, dem sogenannten *Wilcoxon-matched pairs signed ranks*-Test (s. Siegel, „Non Parametric Statistics", S. 75 bis 83). In den meisten Fällen erfolgt eine Angabe der minimalen effektiven Dosis (MED).

Die erfindungsgemässen Verbindungen erhöhen mit 10 bis 50 mg/kg. *p.o.* überraschend stark die Tastendruckrate in der Schockphase, während in der schockfreien Phase bis 100 mg/kg *p.o.* keine Sedierung zu beobachten ist. Die erfindungsgemässen Verbindungen sind somit neue Anxiolytica mit einem grossen Split zwischen angstlösender und sedierender Wirkung. Die Verbindungen weisen eine geringe Toxizität auf. Die $LD_{50}$-Werte (i.p. Maus) liegen im allgemeinen über 1000 mg/kg.

Die erfindungsgemässen Verbindungen und ihre pharmazeutisch verträglichen Salze sind innerhalb eines breiten Dosierungsbereiches wirksam, wobei die jeweils verabreichte Dosis von verschiedenen Faktoren, wie z.B. der jeweils verwendeten Verbindung, dem Zustand, dem Typ und der Grösse des zu behandelnden Säugetieres, abhängt. Die pro Tag erforderliche Dosis liegt bei der Behandlung von erwachsenen Menschen normalerweise innerhalb des Bereiches von 1 bis 60 mg.

Bei der Behandlung von Testtieren, wie Mäusen und Ratten, sind Einzeldosen von 5 bis 50 mg/kg angebracht. Die erfindungsgemässen aktiven Verbindungen und ihre Salze werden normalerweise oral oder durch Injektion verabreicht.

Als Arzneimittel wird mindestens eine erfindungsgemässe Verbindung oder ein Salz davon in Kombination mit einem dafür geeigneten, pharmazeutisch verträglichen Träger verwendet.

Zur Herstellung der erfindungsgemässen pharmazeutischen Mittel wird der aktive Bestandteil in der Regel mit einem Träger gemischt oder mit einem Träger verdünnt oder von einem Träger eingeschlossen, der in Form einer Kapsel, in Form eines Sachets oder in Form eines anderen Behälters vorliegen kann; wenn er als Verdünnungsmittel dient, kann es sich dabei um ein festes, halbfestes oder flüssiges Material handeln, das als Verdünnungsmittel, Hilfsstoff oder Medium für den aktiven Bestandteil (Wirkstoff) dient. Beispiele für geeignete Träger sind Lactose, Dextrose, Saccharose, Sorbit, Mannit, Stärke, Akaziengummi, Calciumphosphat, Alginate, Tragant, Gelatine, Sirup, Methylcellulose, Methyl- und Propylhydroxybenzoat, Talk, Magnesiumstearat oder Mineralöl.

Die erfindungsgemässen pharmazeutischen Mittel können auf an sich bekannte Weise so formuliert werden, dass sie den Wirkstoff nach der Verabreichung an den Patienten schnell, kontinuierlich oder verzögert abgeben.

Je nach Art der Verabreichung können die oben angegebenen pharmazeutischen Mittel zu Tabletten, Kapseln oder Suspensionen für die orale Verwendung und zu Injektionslösungen für die parenterale Verwendung verarbeitet werden.

*Beispiel 1:*

*7-Chlor-5-phenyl-2-phenylcarbamoyloxyamino-3H-1,4-benzodiazepin*

a) 1,43 g 7-Chlor-2-hydroxyamino-5-phenyl-3H-1,4-benzodiazepin werden in 25 ml DMF gelöst und bei Raumtemperatur mit 0,6 ml Phenylisocyanat versetzt. Nach 2 h Rühren bei Raumtemperatur wird die Reaktionslösung in 200 ml Eiswasser gegossen, 30 min nachgerührt, abgesaugt und mit Wasser gut nachgewaschen. Nach Trocknen im Hochvakuum bei 40 bis 50° C

schmilzt die farblose Substanz bei 212 bis 215° C. Ausbeute 1,8 g.

b) 7,5 g 7-Chlor-1,3-dihydro-5-phenyl-2H-1,4-benzodiazepin-2-thion werden in 125 ml Dioxan und 50 ml Dimethylformamid suspendiert und mit 2,2 g Natriumhydrogencarbonat und 1,8 g Hydroxylammoniumchlorid versetzt. Nach 8 h Rühren bei Raumtemperatur (DC-Kontrolle) wird abgesaugt und 2,8 ml Phenylisocyanat zugegeben. Der Reaktionsansatz wird nach 30minütigem Nachrühren in 200 ml Wasser eingetropft und abgesaugt. Umkristallisation aus Ethanol/Dimethylformamid liefert farblose Kristalle vom Schmelzpunkt = Fp. 215 bis 217° C. Ausbeute 4,6 g.

*Beispiel 2:*

*7-Chlor-2-(3-chlorphenylcarbamoyloxyamino)-5-phenyl-3H-1,4-benzodiazepin*

a) 1,5 g 7-Chlor-2-hydroxyamino-5-phenyl-3H-1,4-benzodiazepin werden in 25 ml DMF vorgelegt und mit 0,6 ml 3-Chlorphenylisocyanat versetzt. Nach 2 h Rühren bei Raumtemperatur (DC-Kontrolle) wird der Reaktionsansatz in 200 ml Eiswasser gegossen, 30 min nachgerührt und abgesaugt. Nach Trocknen im Vakuum wird aus Ethanol umkristallisiert. 2,05 farblose Kristalle vom Schmelzpunkt = Fp. 157 bis 159° C. Das Hydrochlorid schmilzt bei 210° C (aus Methanol/DMF) unter Zersetzung.

b) 30 g 7-Chlor-1,3-dihydro-5-phenyl-2H-1,4-benzodiazepin-2-thion werden in 500 ml Dioxan suspendiert und anschliessend mit 17,6 g Natriumhydrogencarbonat und 7,2 g Hydroxylammoniumchlorid versetzt. Durch Zugabe von DMF wird nahezu alles in Lösung gebracht. Nach 8 h Rühren bei Raumtemperatur (DC-Kontrolle) wird abgesaugt und mit 12,8 ml 3-Chlorphenylisocyanat versetzt. Es wird 1 h nachgerührt, mit Wasser versetzt und abgesaugt. Umkristallisation aus Ethanol liefert farblose Kristalle vom Schmelzpunkt = Fp. 158 bis 159° C. Ausbeute 17,4 g.

*Beispiel 3:*

*7-Chlor-2-(4-chlorphenylcarbamoyloxyamino)-5-phenyl-3H-1,4-benzodiazepin*

2 g 7-Chlor-2-hydroxyamino-5-phenyl-3H-1,4-benzodiazepin werden analog zu Versuch 1a mit 1 g 4-Chlorphenylisocyanat umgesetzt. Schmelzpunkt = Fp. 200 bis 203° C (aus Methanol). Ausbeute 2,1 g.

*Beispiel 4:*

*7-Chlor-2-(2-trifluormethylphenylcarbamoyloxyamino)-5-phenyl-3H-1,4-benzodiazepin*

2 g 7-Chlor-2-hydroxyamino-5-phenyl-3H-1,4-benzodiazepin werden analog zu Versuch 1a mit 1 g 2-Trifluormethylphenylisocyanat umgesetzt. Ausbeute: 1,8 g farblose Kristalle vom Schmelzpunkt = Fp. 190° C (aus Methanol/DMF).

Analog zu Beispiel 1 wurden folgende Beispiele (5 bis 7) dargestellt (Tabelle 1).

*Tabelle 1*

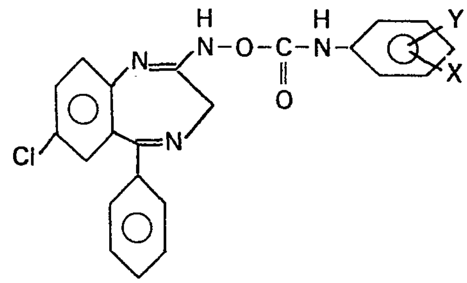

| Beispiele | X | Y | Schmp. (bzw. Zers. p.) |
|---|---|---|---|
| 5 | 3-$CF_3$ | H | 95° C (aus Ethanol) |
| 6 | 4-$CF_3$ | H | 215-218° C (aus Isopropanol) |
| 7 | 2-F | H | 193-195° C (aus Ethanol) |

*Beispiel 8:*

*7-Nitro-5-phenyl-2-phenylcarbamoyloxyamino-3H-1,4-benzodiazepin*

3 g 7-Nitro-1,3-dihydro-5-phenyl-2H-1,4-benzodiazepin-2-thion werden in 52 ml Dioxan und 40 ml Dimethylformamid gelöst. Nach Zugabe von 0,9 g Natriumhydrogencarbonat und 0,75 g Hydroxylammoniumhydrochlorid wird 8 h nachgerührt (DC-Kontrolle), abgesaugt und das Filtrat mit 1 ml Phenylisocyanat versetzt. Es wird 1 h nachgerührt, in Wasser gegossen und das ausfallende Öl abgetrennt, mit Ethanol versetzt und der kristalline Niederschlag abgesaugt.

Ausbeute: 1 g schwach gelbe Kristalle vom Schmelzpunkt = Fp. 182 bis 185° C (unter Zersetzung).

*Beispiel 9:*

*2-(3-Chlorphenylcarbamoyloxyamino)-7-nitro-5-phenyl-3H-1,4-benzodiazepin*

3 g 7-Nitro-1,3-dihydro-5-phenyl-2H-1,4-benzodiazepin-2-thion werden analog zu Beispiel 8 mit 3-Chlorphenylisocyanat umgesetzt und aufgearbeitet. Nach säulenchromatographischer Reinigung fallen 0,6 g gelbe Kristalle vom Schmelzpunkt = Fp. 123 bis 125° C (aus Ethanol) an.

*Beispiel 10:*

*5-(2-Chlorphenyl)-7-nitro-2-phenylcarbamoyloxyamino-3H-1,4-benzodiazepin*

2 g 5-(2-Chlorphenyl)-7-nitro-1,3-dihydro-2H-1,4-benzodiazepin-2-thion analog zu Beispiel 8 0,8 g Natriumhydrogencarbonat und 0,7 g Hydroxylammoniumchlorid umgesetzt. Nach 8 h Rühren bei Raumtemperatur werden 0,8 ml Phenylisocyanat zugegeben, ½ h nachgerührt, mit Wasser gefällt und das anfallende Öl mit Ethanol zur Kristallisation gebracht. Umkristallisation aus Ethanol liefert 2 g gelbe Kristalle vom Schmelzpunkt = Fp. 201 bis 203° C.

*Beispiel 11:*

*5-(2-Chlorphenyl)-2-(3-chlorphenylcarbamoyloxyamino)-7-nitro-3H-1,4-benzodiazepin*

2 g 5-(2-Chlorphenyl)-7-nitro-1,3-dihydro-2H-1,4-benzodiazepin-2-thion werden analog zu Beispiel 10 mit 0,8 ml 3-Chlorphenylisocyanat umgesetzt und wie dort beschrieben aufgearbeitet.

Ausbeute: 0,6 g gelbe Kristalle vom Schmelzpunkt = Fp. 130 bis 135° C (aus Ethanol).

*Beispiel 12:*

*7-Chlor-5-(2-fluorphenyl)-2-phenylcarbamoyloxyamino-3H-1,4-benzodiazepin*

2 g 7-Chlor-5-(2-fluorphenyl)-1,3-dihydro-2H-1,4-benzodiazepin-2-thion werden in 40 ml Dioxan und 20 ml DMF mit 0,6 g Natriumhydrogencarbonat und 0,5 g Hydroxylammoniumchlorid versetzt und 8 h bei Raumtemperatur gerührt. Weitere Zugabe von 0,2 g Natriumhydrogencarbonat und 0,2 g Hydroxylammoniumchlorid, ½ h Rühren bei 50° C und Zugabe von 0,8 ml Phenylisocyanat liefert nach üblicher Aufarbeitung 0,7 g farblose Kristalle vom Schmelzpunkt = Fp. 126 bis 128° C (aus Isopropanol).

Analog zu Beispiel 12 lassen sich die Beispiele 13 bis 15 darstellen:

*Beispiel 13:*

*7-Chlor-2-(3-chlorphenylcarbamoyloxyamino)-5-(2-fluorphenyl)-2H-1,4-benzodiazepin*

Schmelzpunkt = Fp. 194 bis 196° C (aus Ethanol).

*Beispiel 14:*

*7-Chlor-2-(3-chlorphenylcarbamoyloxyamino)-5-(2-chlorphenyl)-2H-1,4-benzodiazepin*

Schmelzpunkt = Fp. 125 bis 137° C (aus Ethanol).

*Beispiel 15:*

*7-Chlor-5-(2-chlorphenyl)-2-phenylcarbamoyloxyamino-2H-1,4-benzodiazepin*

Schmelzpunkt = Fp. 224 bis 226° C (aus Ethanol).

*Beispiel 16:*

*2-(3-Chlorphenylcarbamoyloxyamino)-5-(2-fluorphenyl)-7-nitro-3H-1,4-benzodiazepin*

2,5 g 1,3-Dihydro-5-(2-fluorphenyl)-7-nitro-2H-1,4-benzodiazepin-2-thion werden in 65 ml Dioxan und 30 ml Dimethylformamid gelöst und mit 1,0 g Natriumhydrogencarbonat und 0,88 g Hydroxylammoniumchlorid versetzt. Nach 2 h Rühren bei Raumtemperatur werden noch Natriumhydrogencarbonat und Hydroxylammoniumchlorid zugegeben. Nach noch 2 h weiter wird abgesaugt und 0,58 ml 3-Chlorphenylisocyanat zugegeben. Es wird analog zu Beispiel 10 aufgearbeitet.

Ausbeute: 0,55 g, Schmelzpunkt = Fp. 126 bis 130° C (aus Methanol/Ethanol).

**Patentansprüche** für die Vertragsstaaten BE, CH, DE, FR, GB, IT, LI, LU, NL, SE

1. Carbamoyloxyamino-1,4-benzodiazepine der Formeln I und IA

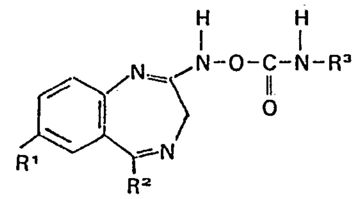

(I)

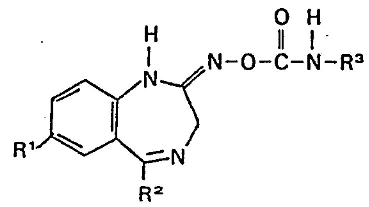

(IA)

und deren physiologisch verträgliche Salze, worin

$R^1$ ein Halogenatom oder eine Nitrogruppe ist,

$R^2$ Phenyl oder ein durch Halogen einfach substituierter Phenylring ist, und

$R^3$ Phenyl oder ein durch Halogen oder Trifluormethyl einfach substituierter Phenylring ist.

2. Verfahren zur Herstellung von Verbindungen gemäss Anspruch 1, dadurch gekennzeichnet, dass man

a) eine Verbindung der allgemeinen Formel II

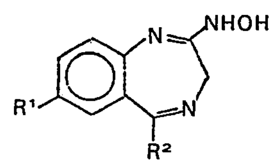

(II)

worin $R^1$ und $R^2$ die in Anspruch 1 genannte Bedeutung haben, mit einem Isocyanat der Formel III

$$O=C=N-R^3 \qquad (III)$$

oder einem reaktionsfähigen Carbaminsäurederivat der Formel IV

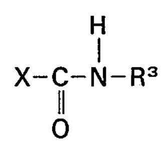

(IV)

umsetzt, worin X für Halogen oder den Phenoxyrest steht und $R^3$ die in Anspruch 1 genannte Bedeutung hat;

b) eine Verbindung der Formel V

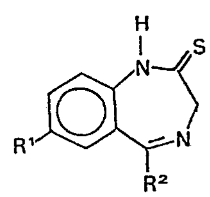

(V)

worin $R^1$ und $R^2$ die in Anspruch 1 genannte Bedeutung besitzen, mit Hydroxylamin bzw. einem Salz des Hydroxylamins, aus dem während der Reaktion die Base freigesetzt wird, zur Reaktion bringt und anschliessend mit einem Isocyanat der Formel III oder einem Carbaminsäurederivat der Formel IV umsetzt; oder

c) eine Verbindung der Formel V mit einem O-acylierten Hydroxylamin der Formel VI

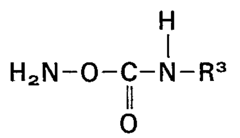
(VI)

worin $R^3$ die in Anspruch 1 genannte Bedeutung hat, umsetzt.

3. 7-Chlor-2-(3-chlorphenylcarbamoyloxyamino)-5-phenyl-3H-1,4-benzodiazepin.

4. 5-(2-Chlorphenyl)-2-(3-chlorphenylcarbamoyloxyamino)-7-nitro-3H-1,4-benzodiazepin.

5. 7-Chlor-5-(2-chlorphenyl)-2-(3-chlorphenylcarbamoyloxyamino)-3H-1,4-benzodiazepin.

6. 2-(3-Chlorphenylcarbamoyloxyamino)-5-(2-fluorphenyl)-7-nitro-3H-1,4-benzodiazepin.

7. 2-(3-Chlorphenylcarbamoyloxyamino)-7-nitro-5-phenyl-3H-1,4-benzodiazepin.

8. 7-Chlor-2-(3-chlorphenylcarbamoyloxyamino)-5-(2-fluorphenyl)-3H-1,4-benzodiazepin.

9. Pharmazeutische Zubereitungen, dadurch gekennzeichnet, dass sie eine Verbindung gemäss Anspruch 1 und einen pharmazeutisch verträglichen Träger enthalten.

**Patentanspruch** für den Vertragsstaat: AT

Verfahren zur Herstellung von Carbamoyloxyamino-1,4-benzodiazepinen der Formeln I und IA

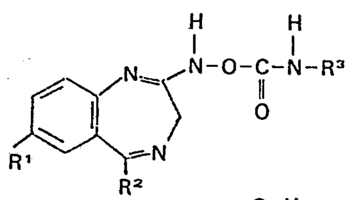
(I)

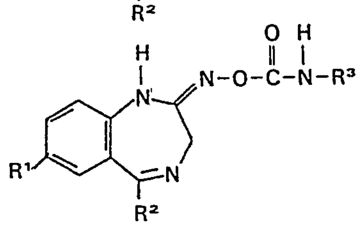
(IA)

und deren physiologisch verträgliche Salze, worin

$R^1$ ein Halogenatom oder eine Nitrogruppe ist,

$R^2$ Phenyl oder ein durch Halogen einfach substituierter Phenylring ist,

$R^3$ Phenyl oder ein durch Halogen oder Trifluormethyl einfach substituierter Phenylring ist, dadurch gekennzeichnet, dass man

a) eine Verbindung der allgemeinen Formel II

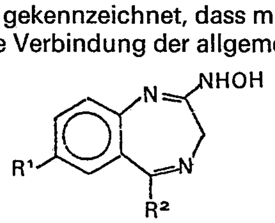
(II)

worin $R^1$ und $R^2$ die obengenannte Bedeutung haben, mit einem Isocyanat der Formel III

$$O{=}C{=}N{-}R^3 \qquad (III)$$

oder einem reaktionsfähigen Carbaminsäurederivat der Formel IV

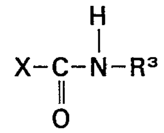
(IV)

umsetzt, worin X für Halogen oder den Phenoxyrest steht und $R^3$ die obengenannte Bedeutung hat;

b) eine Verbindung der Formel V

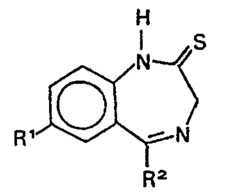
(V)

worin $R^1$ und $R^2$ die obengenannte Bedeutung besitzen, mit Hydroxylamin bzw. einem Salz des Hydroxylamins, aus dem während der Reaktion die Base freigesetzt wird, zur Reaktion bringt und anschliessend mit einem Isocyanat der Formel III oder einem Carbaminsäurederivat der Formel IV umsetzt; oder

c) eine Verbindung der Formel V mit einem O-acylierten Hydroxylamin der Formel VI

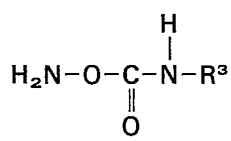
(VI)

worin $R^3$ die obengenannte Bedeutung hat, umsetzt.

**Claims** for the Contracting States BE, CH, DE, FR, GB, IT, LI, LU, NL, SE

1. Carbamoyloxyamino-1,4-benzodiazepines of the formulae I and IA

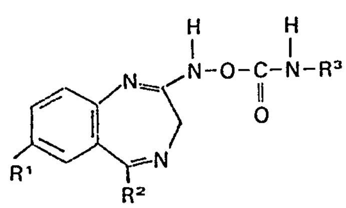
(I)

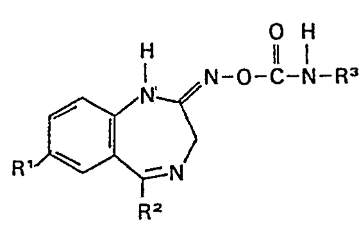
(IA)

and physiologically tolerated salts thereof, wherein $R^1$ is a halogen atom, or a nitro group; $R^2$ is phenyl, or a phenyl ring which is mono-substituted by halogen; and $R^3$ is phenyl or a phenyl ring which is mono-substituted by halogen or trifluoromethyl.

2. A process for the preparation of compounds as claimed in Claim 1, in which

(*a*) a compound of the general formula II

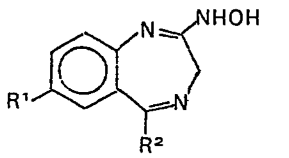

(II)

wherein $R^1$ and $R^2$ have the meaning mentioned in Claim 1, is reacted with an isocyanate of the formula III

$$O{=}C{=}N{-}R^3 \qquad (III)$$

or with a carbamic acid derivative of the formula IV

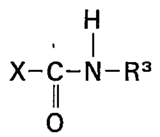

(IV)

which is capable of reaction, wherein X represents halogen or the phenoxy radical and $R^3$ has the meaning mentioned in Claim 1;

(*b*) a compound of the formula V

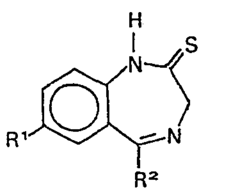

(V)

wherein $R^1$ and $R^2$ have the meaning mentioned in Claim 1, is reacted with hydroxylamine, or a salt of the hydroxylamine from which the base is liberated during the reaction, and then with an isocyanate of the formula III or a carbamic acid derivative of the formula IV; or

(*c*) a compound of the formula V is reacted with an O-acylated hydroxylamine of the formula VI

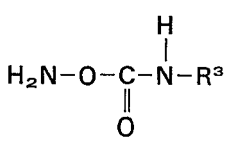

(VI)

wherein $R^3$ has the meaning mentioned in Claim 1.

3. 7-Chloro-2-(3-chlorophenylcarbamoyloxyamino)-5-phenyl-3H-1,4-benzodiazepine.

4. 5-(2-chlorophenyl)-2-(3-chlorophenylcarbamoyloxyamino)-7-nitro-3H-1,4-benzodiazepine.

5. 7-Chloro-5-(2-chlorophenyl)-2-(3-chlorophenylcarbamoyloxyamino)-3H-1,4-benzodiazepine.

6. 2-(3-Chlorophenylcarbamoyloxyamino)-5-(2-fluorophenyl)-7-nitro-3H-1,4-benzodiazepine.

7. 2-(3-Chlorophenylcarbamoyloxyamino)-7-nitro-5-phenyl-3H1,4-benzodiazepine.

8. 7-Chloro-2-(3-chlorophenylcarbamoyloxyamino)-5-(2-fluorophenyl)-3H-1,4-benzodiazepine.

9. Pharmaceutical formulations which contain a compound as claimed in Claim 1 and a pharmaceutically tolerated carrier.

**Revendications** pour les Etats contractants BE, CH, DE, FR, GB, IT, LI, LU, NL, SE

1. Carbamoyloxyamino-1,4-benzodiazépines de formules I et IA

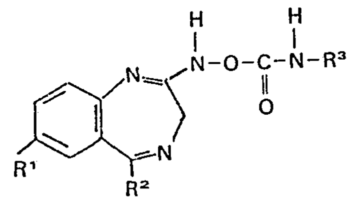

(I)

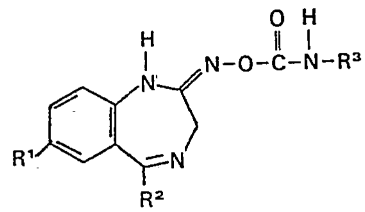

(IA)

et leurs sels physiologiquement acceptables, dans lesquelles

$R^1$ représente un atome d'halogène ou un groupe nitro,

$R^2$ représente un groupe phényle ou un noyau phényle monosubstitué par un halogène,

$R^3$ représente un groupe phényle ou un noyau phényle monosubstitué par un halogène ou par un groupe trifluorométhyle.

2. Procédé pour la préparation de composés selon la revendication 1, caractérisé en ce qu'il consiste:

a) à faire réagir un composé de formule générale II

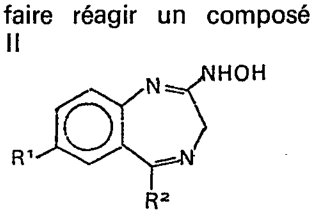

(II)

dans laquelle $R^1$ et $R^2$ ont les significations indiquées dans la revendication 1, avec un isocyanate de formule III

$$O{=}C{=}N{-}R^3 \qquad (III)$$

ou avec un dérivé réactif de l'acide carbamique de formule IV

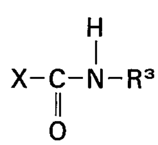

(IV)

dans laquelle X représente un halogène ou le groupe phénoxy et $R^3$ a la signification indiquée dans la revendication 1;

b) à faire réagir un composé de formule V

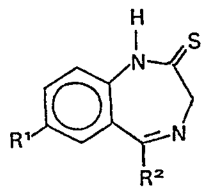

(V)

dans laquelle $R^1$ et $R^2$ ont les significations indiquées dans la revendication 1, avec l'hydroxylamine ou un sel d'hydroxylamine, dont la base est libérée pendant la réaction, puis à faire réagir avec un isocyanate de formule III ou un dérivé de l'acide carbamique de formule IV; ou

c) à faire réagir un composé de formule V avec une hydroxylamine O-acylée de formule VI

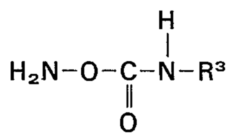

(VI)

dans laquelle $R^3$ a la signification indiquée dans la revendication 1.

3. La 7-chloro-2-(3-chlorophénylcarbamoyloxyamino)-5-phényl-3H-1,4-benzodiazépine.

4. La 5-(2-chlorophényl)-2-(3-chlorophénylcarbamoyloxyamino)-7-nitro-3H-1,4-benzodiapézine.

5. La 7-chloro-5-(2-chlorophényl)-2-(3-chlorophénylcarbamoyloxyamino)-3H-1,4-benzodiazépine.

6. La 2-(3-chlorophénylcarbamoyloxyamino)-5-(2-fuorophényl)-7-nitro-3H-1,4-benzodiazépine.

7. La 2-(3-chlorophénylcarbamoyloxyamino)-7-nitro-5-phényl-3H-1,4-benzodiazépine.

8. La 7-chloro-2-(3-chlorophénylcarbamoyloxyamino)-5-(2-fluorophényl)-3H-1,4-benzodiazépine.

9. Compositions pharmaceutiques, caractérisées en ce qu'elles contiennent un composé selon la revendication 1 et un véhicule pharmaceutiquement acceptable.

**Claim** for the Contracting State: AT

A process for the preparation of carbamoyloxyamino-1,4-benzodiazepines of the formulae I and IA

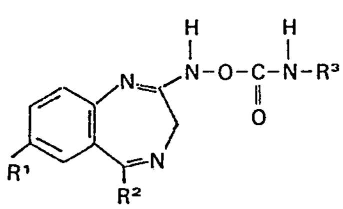

(I)

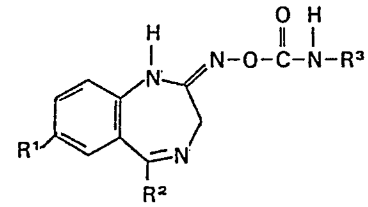

(IA)

and the physiologically tolerated salts thereof, wherein $R^1$ is a halogen atom, or a nitro group; $R^2$ is phenyl or a phenyl ring which is mono-substituted by halogen; and $R^3$ is phenyl or a phenyl ring which is mono-substituted by halogen or trifluoromethyl, in which

(*a*) a compound of the general formula II

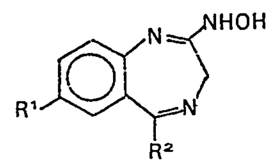

(II)

wherein $R^1$ and $R^2$ have the meaning mentioned above, is reacted with an isocyanate of the formula III

$$O{=}C{=}N{-}R^3 \quad (III)$$

or with a carbamic acid derivative of the formula IV

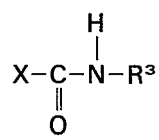

(IV)

which is capable of reaction, wherein X represents halogen or the phenoxy radical and $R^3$ has the meaning mentioned above;

(*b*) a compound of the formula V

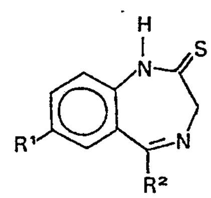

(V).

wherein $R^1$ and $R^2$ have the meaning mentioned above, is reacted with hydroxylamine, or a salt of the hydroxylamine from which the base is liberated during the reaction, and then with an isocyanate of the formula III or a carbamic acid derivative of the formula IV; or

(*c*) a compound of the formula V is reacted with an O-acylated hydroxylamine of the formula VI

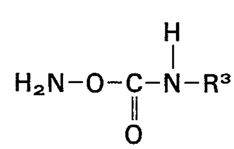

(VI)

wherein $R^3$ has the meaning mentioned above.

**Revendication** pour l'Etat contractant: AT

Procédé pour la préparation de carbamoyloxyamino-1,4-benzodiazépines de formules I et IA

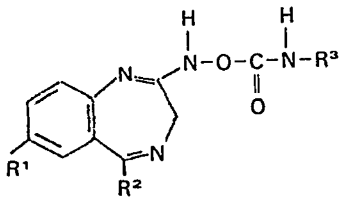

(I)

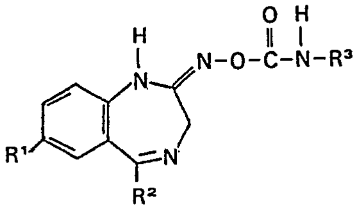

(IA)

et de leurs sels physiologiquement acceptables, dans lesquelles

$R^1$ représente un atome d'halogène ou un groupe nitro,

$R^2$ représente un groupe phényle ou un noyau phényle monosubstitué par un halogène,

$R^3$ représente un groupe phényle ou un noyau phényle monosubstitué par un halogène ou un groupe trifluorométhyle, lequel procédé est caractérisé en ce qu'il consiste

a) à faire réagir un composé de formule générale II

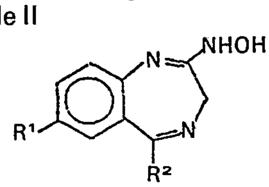

(II)

dans laquelle $R^1$ et $R^2$ ont les significations indiquées ci-dessus, avec un isocyanate de formule

$$O=C=N-R^3 \qquad (III)$$

ou avec un dérivé réactif de l'acide carbamique de formule IV

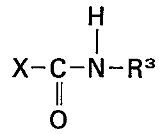

(IV)

dans laquelle X représente un halogène ou le groupe phénoxy et $R^3$ a la signification indiquée ci-dessus;

b) à faire réagir un composé de formule V

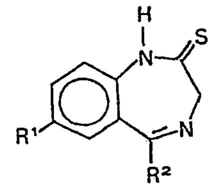

(V)

dans laquelle $R^1$ et $R^2$ ont les significations indiquées ci-dessus, avec l'hydroxylamine ou un sel d'hydroxylamine, dont la base est libérée au cours de la réaction, puis à faire réagir avec un isocyanate de formule III ou un dérivé de l'acide carbamique de formule IV; ou

c) à faire réagir un composé de formule V avec une hydroxylamine O-acylée de formule VI

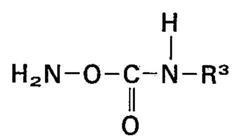

(VI)

dans laquelle $R^3$ a la composition indiquée ci-dessus.